# EUROPEAN PATENT APPLICATION

(11) **EP 3 345 655 A1**
(43) Date of publication of application: **11.07.2018**
(21) Application number: 18156400.6
(22) Date of filing: 02.06.2009
(51) Int. Cl.: A61Q 11/00, A61K 8/86, A61K 8/73, A61K 8/81

(54) **ORAL CARE COMPOSITIONS AND METHODS**

(30) Priority: 04.06.2008 US 58609 P
(62) Divisional of application: 09759191.1
(71) Applicant: The Procter & Gamble Company, Cincinnati, OH 45202 (US)
(72) Inventor: BAIG, Arif Ali, Cincinnati, OH 45202 (US); BRAS, Rafael Edmundo, Cincinnati, OH 45202 (US); CERDA, Luisa Navarro, Cincinnati, OH 45202 (US); HAMERSKY, Mark William, Cincinnati, OH 45202 (US); LEONARD, Robert Scott, Cincinnati, OH 45202 (US); MEDEIROS, Franco Silva, Cincinnati, OH 45202 (US); RAJAIAH, Jayanth, Cincinnati, OH 45202 (US); SMITH, Steven Daryl, Cincinnati, OH 45202 (US)
(74) Representative: Joos, Uta Susanne

(57) **Abstract**

The present invention relates to oral care compositions that are not denture adhesives. The oral care compositions include an adhesive component and a whitening agent as oral care active, and are combined with petrolatum as water insoluble component. The present invention is also directed to methods relating to the oral care compositions.

## Description

### TECHNICAL FIELD

This invention relates to oral care compositions and in particular to improved oral care methods and compositions which include an adhesive component and an oral care active combined with a viscosity index improver and/or a water insoluble component.

### BACKGROUND OF THE INVENTION

Oral care compositions are used in many areas of life from daily maintenance of the oral cavity to the treatment of conditions in the oral cavity or administration of actives in the oral cavity. Oral care actives used for treatment are usually placed within or on a water soluble carrier and applied to the oral cavity. Unfortunately, many such vehicles erode before treatment can be completed due to over hydration of the carrier and active by saliva and other liquids in the mouth. As such, there is a need for improved oral care compositions.

### SUMMARY OF THE INVENTION

According to one embodiment, the present invention is directed to an oral care composition, comprising an adhesive component, a viscosity index improver, and an oral care active, wherein the oral care composition is adapted to be applied to at least a portion of the oral cavity.

In another embodiment, the present invention is directed to an oral care composition, comprising: a) from about 30% to about 50 % of an adhesive component; b) from about 40% to about 60 % of a viscosity index improver comprising microcrystalline wax, polyethylene, rubber, elastomers, or a combination thereof; and c) an oral care active, wherein the composition is preformed and adapted to be applied to at least a portion of the oral cavity.

In an additional embodiment, the present invention is directed to an oral care composition, comprising: a) an adhesive component, b) a water insoluble component, and c) an oral care active, wherein the composition is adapted to be applied to at least a portion of the oral cavity.

These and other embodiments of the present invention will be more fully understood in light of the detailed description below.

### DETAILED DESCRIPTION OF THE INVENTION

A detailed description of embodiments of the present invention is given below.

### DEFINITIONS

The abbreviations listed here have the following meanings as used herein: "cm" means centimeter, "mm" means millimeter, "g" means gram, "P" means Pascal, "s" means second, "Ps" means Pascal - second, and "oz" means ounce.

The term "oral cavity" as used herein refers to any surface inside the mouth of an animal or human, including but not limited to, cheeks, teeth, gums, lips, gingiva, tongue, palate, bridges, crowns, restorations, varnishes, sealants, fillings, implants, orthodontic appliances, etc.

The term "oral care composition" used herein refer to compositions which are used within the oral cavity, excluding denture adhesives.

The term "oral care active" as used herein refers to actives which can be used to treat or help prevent a condition of the oral cavity or which can be administered through the oral cavity.

The term "viscosity index improver" as used herein refers to a material which makes the viscosity and/or rheology of a material into which it is incorporated more stable as its temperature is increased over a defined range. In the case of oral care products, the defined range is between about 25°C and about 60°C.

The term "dispensed/dispensable from a tube" as used herein refers to a composition which can be dispensed from a small tube under manual pressure. A small tube is made of a foil laminate, is about 3.5 inches long, about 0.48 inches wide, and holds about 0.25 oz of product. The internal diameter of the nozzle on the small tube is about 0.19 inches and the nozzle length is about 0.38 inches. An example of a small tube is a 0.25 oz sample size tube which is supplied by Alcan Corporation as stock item no. 2293.

The term "preformed" as used herein refers to an oral care composition which has been formed into a sheet suitably shaped to fit onto an oral cavity.

By "safe and effective amount", as used herein, is meant an amount of an agent high enough to significantly (positively) modify the condition to be treated or positively modify the benefit sought, but low enough to avoid serious side effects (at a reasonable benefit/risk ratio), within the scope of sound medical/dental judgment.

The term "AVE/MA" as used herein refers to alkyl vinyl ether-maleic acid or anhydride copolymer. The term "mixed salts", as used herein, refers to salts of polymers where at least 2 different cations are mixed on the same polymer with each other or with other salts.

The term "toxicologically-acceptable", as used herein, is used to describe materials that are suitable in their toxicity profile for administration to humans and/or animals.

The term "non-aqueous", as used herein, means the composition is substantially free of added water. Substantially free means that no free water is added to the composition, but the composition may contain about 5% or less of water which comes in as part of other components.

The term "water-insoluble" as used herein refers to a material that, when exposed to water, does not dissolve, but may disperse to varying degrees.

The term "bioerodible" as used herein means that the composition, when exposed to water or saliva, will erode over time due to physical and/or chemical action. The composition may erode completely or substantially, however ultimately the composition will lose its original form and/or integrity.

Unless otherwise noted, the term "melting point" as used herein refers to the Drop Melting Point which is the temperature at which the material becomes sufficiently fluid to drop from the thermometer used in making the determination under prescribed conditions as listed in ASTM D-127. ASTM D-3954 is an alternate way to measure melting point.

Unless otherwise noted, the term "derivative" as used herein refers to when the primary polymeric backbone is left unchanged, but the side groups/chains and/or end groups are changed.

As used herein, the term "silicone" refers to siloxane polymers based on a structure of alternate silicon and oxygen atoms with various organic radicals attached to the silicon.

All other percentages used herein are by weight of the composition unless otherwise indicated. All measurements referred to herein are made at 25°C unless otherwise specified.

### ORAL CARE COMPOSITIONS AND METHODS

Historically, oral care products like dentifrices and rinses have utilized water soluble components as a base to deliver various actives to the oral cavity. This approach has several disadvantages. For example, the water soluble bases and actives are easily dissolved away in saliva and the choice of oral care actives are limited to those that do not interact with the water soluble components or with each other since the actives are dissolved in the water soluble base. Unlike conventional oral care products, denture adhesive compositions use water insoluble components to deliver adhesive particles to the oral cavity. It has now been surprisingly discovered that water insoluble components, like those used in denture adhesives, can be used in oral care compositions to give improved delivery of oral care actives to the oral cavity as the oral care actives are no longer required to be dissolved in the water soluble base.

Further, it has been discovered that a viscosity index improver can increase the beneficial effects of the water insoluble component and has additional benefits standing on its own. Historically, viscosity index improver was a term associated with the lubricant industry. The viscosity of a lubricant is closely related to its ability to reduce friction. The most desirable lubricant is one which will allow the easiest movement of two surfaces while still forcing the two moving surfaces apart, because this results in the lowest friction. However, as the viscosity of liquids tends to decrease as the temperature increases, many lubricants which work at lower temperatures are not thick enough to work at higher temperatures and those that are thick enough at the higher temperatures have a tendency to be too thick to work at the lower temperatures. The best lubricants will not vary much in viscosity over a desired temperature range and therefore will perform well throughout.

In order to better predict the range of temperatures at which a lubricant would work, the Society of Automotive Engineers established the Viscosity Index. The Viscosity Index highlights how a lubricant's viscosity changes with variations in temperature. The Viscosity Index shows the viscosity of materials at an arbitrary "low" temperature of 100 °Fahrenheit (40 °C) and an arbitrary "high" temperature of 210 °F (100 °C).

After understanding the properties of lubricants over the set temperature ranges, it was discovered that adding certain types of compounds to the lubricants would make the viscosity of the lubricants more consistent through a broader temperature range. Thus, there was less of a decrease in the viscosity of the lubricant at the higher temperatures. Having a higher viscosity at the higher temperature allowed the lubricants to work better at the higher temperatures. The materials added to increase the viscosity at higher temperatures were defined as viscosity index improvers.

It has surprisingly been discovered that application of that principal also has relevance to oral care compositions. In general, oral care compositions can be made up of a myriad of materials based on the end use. For those which are intended to deliver an active to the oral cavity through adhesion, they generally comprise an adhesive component and a carrier. During
use, the moisture in the saliva penetrates through the carrier and hydrates the adhesive component and the active. This makes the adhesive component sticky to the mucosal tissue and other oral surfaces. The viscosity of the oral care composition contributes to the speed at which the adhesive component can be hydrated. The amount of hydration is influenced by, the amount of adhesive component, the amount of water insoluble vehicle, and the viscosity of the water insoluble vehicle, all three of which contribute to the overall viscosity of the oral care composition. The viscosity of the oral care composition contributes to the rate and/or amount of hydration of the adhesive component. Over time, excess hydration due to excess saliva and/or liquids can lead to loss of some of the adhesive, thereby weakening it, and loss of the active. Temperature-resistance of the viscosity imparted by the viscosity index improver results in resistance to excess hydration, which in turn results in more adhesive and active being retained over time. This leads to extended and improved performance of the oral care compositions.

The temperature range most relevant for oral care compositions is from room temperature (25°C) which deals with the viscosity of the oral care composition in the dispenser (tube or package, for example) to 40°C which deals with the viscosity of the oral care composition in the mouth. While the temperatures in the mouth can reach upward of 60°C when drinking a hot beverage, looking at the behavior of the compositions at 40°C tends to be a good predictor of having increased beneficial properties at 60°C as well. Thus, viscosity index improvers relevant for oral care compositions will make the viscosity more stable over the range of functional temperatures (i.e. 25°C to 60°C).

Thus, the use of viscosity index improvers alone or in combination with a water insoluble component will improve the hydration characteristics of an oral care composition and/or compatibility with oral care actives and thus provide an improved performance. In light of the above, in one embodiment, oral care compositions according to the present invention comprise an adhesive component and an oral care active combined with a water insoluble component and/or a viscosity index improver.

### ADHESIVE COMPONENT

The present invention comprises a safe and effective amount of an adhesive component, generally at a level of from about 5% to about 99% by weight of the composition. In other embodiments, the adhesive component is in the range of from about 5%, 10%, 15%, 20%, 25%, 30%, 35%, 40% to about 50%, 55%, 60%, 65%, 70%, 75%, or any combination thereof. In one particular embodiment, the adhesive component is in an amount from about 10.0% to about 60.0%.

In general, adhesive components are hydrophilic particles that become sticky when activated by moisture or are hydrophilic liquids. For those that activate with moisture, moisture can be present, for example, in the oral care composition itself as well as in the oral cavity of the user. In varying embodiments, the adhesive components herein are mucoadhesive, hydrophilic, water soluble, have the property of swelling upon exposure to moisture, or any combination thereof.

In one embodiment the adhesive component is selected from the group consisting of: glycerin, polyoxamer, sorbitol, polyox, carbomer, polyacrylamides, polypeptides, natural gums; synthetic polymeric gums; AVE/MA; AVE/MA/IB; copolymers of maleic acid or anhydride and ethylene, styrene, and/or isobutylene, polyacrylic acid and/or polyacrylates thereof; polyitaconic acid, mucoadhesive polymers; water-soluble hydrophilic colloids; saccharide; cellulose; their derivatives, and mixtures thereof. Examples of such materials include karaya gum; guar gum; gelatin; algin; sodium alginate; tragacanth; chitosan; acrylamide polymers; carboxypolymethylene; polyvinyl alcohol; polyamines; polyquarternary compounds; polyvinylpyrrolidone or its copolymers; cationic polyacrylamide polymers; salts and mixed salts of AVE/MA; polymeric acids, polymeric salts, and copolymers thereof; polyitaconic acid salts; polyhydroxy compounds; their derivatives; and mixtures thereof.

In another embodiment, the adhesive component is selected from the group consisting of: cellulose, cellulose derivatives (such as methylcellulose, carboxymethylcellulose, hydroxyethylcellulose, hydroxypropylcellulose, hydroxy-propylmethylcellulose, corn starch, and mixtures thereof), starch, starch derivatives, saccharide, saccharide derivatives, polyethylene oxides, polyethylene glycols, polyvinyl alcohols, carrageenan, alginates, karaya gums, xanthan gums, guar gums, gelatins, algins, tragacanth, chitosan, acrylamide polymers, carboxypolymethylenes, polyamines, poly quaternary compounds, polyvinylpyrrolidone, AVE/MA, salts of AVE/MA, mixed salts of AVE/MA, polymeric acids, polymeric salts, polyhydroxy compounds, and mixtures thereof.

In one embodiment, the adhesive component is a salt of AVE/MA. In another embodiment the adhesive component comprises a mixed salt of AVE/MA. In one embodiment, the adhesive component comprises a calcium and zinc mixed salt of AVE/MA. In yet another embodiment, the adhesive component is selected from the group consisting of mixed salts of
AVE/MA, cellulose derivatives, and combinations thereof. In another embodiment, the adhesive component comprises AVE/MA, salts of AVE/MA, mixed salts of AVE/MA, sodium carboxymethylcellulose, or combinations thereof. In one embodiment, the adhesive component comprises a combination of a mixed salt of AVE/MA and carboxymethylcellulose.

### WATER INSOLUBLE COMPONENT

In some embodiments, the present composition comprises a safe and effective amount of a water insoluble component. In one embodiment this component is present by weight of the composition at an amount of from about 1%, 2, 5, 10, 20, 25, 30, 35 to about 45, 50, 60, 70, 90%, or any combination thereof. In additional embodiments the water insoluble component is present at an amount from about 20% to about 70%, from about 25% to about 60%, or from about 35% to about 60% by weight of the composition. In yet another embodiment the water insoluble component is substantially non-swellable in water. In some embodiments, the non-swellable water insoluble component swells less than about 10%, 5%, 2%, or 1% in water.

In one embodiment, the water insoluble component is selected from the group consisting of: natural wax, synthetic wax, petrolatum, polyvinyl acetate, natural oils, synthetic oils, fats, silicone, silicone derivatives, dimethicone, silicone resins, hydrocarbons, hydrocarbon derivatives, essential oils, caprilic/capric triglycerides, polybutene, oleic acid, stearic acid, and mixtures thereof. In a further embodiment, the water insoluble component comprises petrolatum, polyvinyl acetate, natural oils, synthetic oils, fats, silicone, silicone derivatives, dimethicone, silicone resins, hydrocarbons, hydrocarbon derivatives, polybutene, oleic acid, stearic acid, essential oils, caprilic/capric triglycerides, or combinations thereof. In an additional embodiment, the water insoluble component is substantially free of petrolatum.

Examples of natural oils include, but are not limited to, vegetable oils (ex. corn oil), soy bean oils, cottonseed oils, palm oils, coconut oils, mineral oils, animal oils (ex. fish oils), etc. Examples of synthetic oils include, but are not limited to, silicone oils, etc. In one embodiment, the water insoluble component comprises a natural oil. In a further embodiment, the natural oil comprises mineral oil. In one embodiment, mineral oil is present in the composition at an amount from about 30% to about 50% and in another embodiment, from about 35% to about 45%.

In some embodiments, the water-insoluble component is a wax. In one embodiment the water insoluble component is a natural or synthetic wax. In varying embodiments, wax is present in an amount from about 1, 2, 5, 8, 10, 15, 20, 40% to about 10, 20, 30, 40, 50, 60, 80% or any combination thereof.

### VISCOSITY INDEX IMPROVERS

As discussed previously, viscosity index improvers make the viscosity of the oral care composition more stable over the range of functional temperatures (i.e. about 25°C to about 60°C). It is believed that another mechanism also contributes to the improved properties of oral care compositions comprising viscosity index improvers. Without being limited by theory, it is believed that at least some improved properties arise when at least some of the particles of an adhesive component are at least partially coated or surrounded by a viscosity index improver. In fact, it has been surprisingly discovered that in at least some embodiments of the present invention, a viscosity index improver, microcrystalline wax for example, can at least partially coat the particles of an adhesive component. This is especially seen when the adhesive composition is made by heating up to or beyond the melting point of the viscosity index improver and then cooled to room temperature. In some embodiments, the viscosity index improver can coat the particles of the adhesive component by solidifying or crystallizing within the pores and/or crevices of particles of the adhesive component.

In some instances, the coating/surrounding of the adhesive component by the viscosity index improver functions as a physical barrier to protect the adhesive particles, for example, from being washed out due to incomplete hydration, excess hydration (from saliva or drinks), change in mouth temperature (ex. due to drinking a hot beverage like coffee), and/or chewing. This can also lead to a better utilization and optimization of the adhesive component which leads to a better performance.

Aside from understanding the general principal of viscosity index improvers, another way to determine whether a material would work as a viscosity index improver in an oral care composition is to look at the instant viscosity ratio. The instant viscosity ratio measures the ratio of the viscosities of the prototype sample at room temperature (25°C) and at an elevated temperature (40°C). The present compositions tend to have a viscosity that is higher at elevated temperatures than those compositions without a viscosity index improver. This is important because the oral care composition is placed into the mouth of a user which has a temperature generally higher than that of room temperature. Additionally, the temperature of a user's mouth can also be increased when ingesting hot beverages. The ability to maintain a higher viscosity at these higher temperatures contributes to better hold and less loss of the oral care composition during use.

The instant viscosity ratio can be measured as outlined below. In one embodiment, the instant viscosity ratio is greater than about 0.25. In another embodiment, the instant viscosity ratio is from about 0.25 to about 1.0. In additional embodiments, the instant viscosity ratio is from about 0.25, 0.3, 0.4, 0.6, 0.7 to about 0.3, 0.4, 0.5, 0.8, 1.0, or any combination thereof. In a further embodiment, the instant viscosity ratio is from about 0.3 to about 0.8. In other embodiments, the instant viscosity ratio is from about 0.3 to about 0.6 or from about 0.3 to about 0.5.

Some examples of viscosity index improvers include polymethacrylates, olefin copolymers, hydrogenated styrene-diene copolymers, styrene polyesters, rubber, polyvinylchloride, nylon, fluorocarbon, polyurethane prepolymer, polyethylene, polystyrene, polypropylene, cellulosic resins, acrylic resins, microcrystalline wax, elastomers, poly(n-butyl vinyl ether), poly(styrene-co-maleic anhydride), poly(alkyl fumarate co-vinyl acetate), alkylated polystyrene, poly(t-butyl styrene), or combination thereof.

Examples of polymethacyrlates include, for example, polyacrylate-co-methacrylate, polymethacrylate-co-styrene, or combinations thereof. Examples of elastomers include, for example, hydrogenated styrene-co-butadiene, hydrogenated styrene-co-isoprene, ethylene-ethylene-propylene polymer, ethylene-propylene polymer, styrene-ethylene-ethylene-propylene-styrene polymer or combinations thereof. An example of a rubber includes hydrogenated polyisoprene. Other examples of viscosity index improvers can be found in "Chemistry and Technology of Lubricants," Chapman and Hall (2nd Ed. 1997).

In one embodiment, the viscosity index improver is selected from the group consisting of polymethacrylates, olefin copolymers, hydrogenated styrene-diene copolymers, styrene polyesters, and combinations thereof. In another embodiment, the viscosity index improver is selected from the group consisting of rubber, polyvinylchloride, nylon, fluorocarbon, polyurethane prepolymer, polyethylene, polystyrene, polypropylene, cellulosic resins, acrylic resins, microcrystalline wax, elastomers, and combinations thereof. In an additional embodiment, the viscosity index improver comprises microcrystalline wax, polyethylene, rubber, elastomers, or a combination thereof.

In another embodiment, the viscosity index improver is polyethylene, such as A-C 1702 and A-C 6702 made by Honeywell. In another embodiment, the viscosity index improver is substantially free of amorphous polyethylene having a molecular weight of at least about 80,000. In an additional embodiment, when the viscosity index improver consists of a polyethylene having an average molecular weight of from about 1000 to about 21,000 then the adhesive component is substantially free of a mixed partial salt of a lower AVE/MA salt of calcium and alkali cations selected from the group consisting of sodium, potassium, and quaternary ammonium cations.

In another embodiment, the viscosity index improver comprises microcrystalline wax. In one embodiment, the microcrystalline wax is refined and/or substantially pure. In an additional embodiment, petrolatum does not contribute the microcrystalline wax. In another embodiment, the microcrystalline wax has a melting point ranging from about 50°C to about 100°C. In further embodiments, the microcrystalline wax has a melting point ranging from about 50°C, 55°C, 60°C, 65°C, 70°C to about 70 °C, 75 °C, 80 °C, 85 °C, 90 °C, 95 °C, 100°C, or any combination thereof. In one particular embodiment, the microcrystalline wax has a melting point ranging from about 75°C to about 85°C. In another embodiment the microcrystalline wax is manufactured by Crompton, Sonneborn (Witco) and referred to and sold under the trademark Mutiwax®W-835.

In some embodiments, viscosity index improvers are used in an amount from about 0.001% to about 90.0%. In varying embodiments, the viscosity index improvers are present in an amount from about 1%, 2, 5, 10, 15, 20, 30, 40 to about 10, 15, 20, 30, 40, 50, 60, 70, 80, 90%, or any combination thereof. In one embodiment, the viscosity index improver is from about 40% to about 60%, when the oral care composition is preformed. In one embodiment, the viscosity index improver is from about 1.0% to about 15.0% when the oral care composition can be dispensed from a tube. In one embodiment, the viscosity index improver is water insoluble and/or non-swellable in water.

### MISCELLANEOUS ADDITIVES

### Plasticizing Agent

The compositions of the present invention may also optionally comprise a safe and effective amount of one or more toxicologically-acceptable plasticizers. In varying embodiments the level of the plasticizing agent ranges from about 0.01% to about 40%, from about 1% to about 10%, or from about 2% to about 5% by weight of the composition.

### Gellant Agents

The compositions of the present invention may also optionally comprise a safe and effective amount of one or more toxicologically-acceptable gellants. In varying embodiments, the level of the gellant agent ranges from about 0.01% to about 40%, from about 1% to about 10%, or from about 2% to about 5%, by weight of the composition.

### Oral Care Actives

The oral care compositions may also comprise one or more oral care actives. Oral care actives may be present at a level of from about 0.1%, 0.5, 1, 5, 10, 15, 20, 25, 30, to about 0.5, 1, 3, 5, 10, 15, 20, 30, 50, 70%, or any combination thereof. Oral care actives include, for example, antimicrobial agents such as iodine, triclosan, peroxides, sulfonamides, bisbiguanides, or phenolics; antibiotics such as tetracycline, neomycin, kanamycin, metronidazole, cetylpyridinium chloride, domiphen bromide, or clindamycin; anti-inflammatory agents such as aspirin, acetaminophen, naproxen and its salts, ibuprofen, ketorolac, flurbiprofen, indomethacin, eugenol, or hydrocortisone; dentinal desensitizing agents such as potassium nitrate, strontium chloride or sodium fluoride; fluorides such as sodium fluoride, stannous fluoride, MFP (monofluorophosphate); anesthetic agents such as lidocaine or benzocaine; whitening agents such as peroxide; anti-fungals such as those for the treatment of *candida albicans*; insulin; steroids; herbal and other plant derived remedies; and baking soda. Other suitable oral care actives are discussed in the Physicians Desk Reference 62nd Ed., 2008 and the Physicians Desk Reference for non-prescription drugs, dietary supplements, and herbs, 29th Ed.

According to one embodiment, the active is selected from the group consisting of: anti-calculus agent, fluoride ion source, stannous ion source, whitening agent, antimicrobial agent, anti-plaque agent, anti-stain agent, anti-deposition agent, anti-gingivitis, anti-tartar, anti-periodontitis, anti-sensitivity, anti-cavity, anti-inflammatory agent, nutrients, antioxidants, anti-viral agent, anti-fungal agent, analgesic agent, anesthetic agent, H-2 antagonist, and combinations thereof.

In another embodiment, the oral care active may also include flavors, fragrances, and/or sensates (ex. warming or cooling agents). Suitable oral care actives in this group include, for example, menthol, wintergreen oil, peppermint oil, spearmint oil, leaf alcohol, clove bud oil, anethole, methyl salicylate, eucalyptol, cassia, 1-8 menthyl acetate, sage, eugenol, parsley oil, oxanone, alpha-irisone, marjoram, lemon, orange, propenyl guaethol, cinnamon, vanillin, thymol, linalool, cinnamaldehyde glycerol acetal, their derivatives, and mixtures thereof. In one embodiment, the active is an aromatic such as camphor, eucalyptus oil, and aldehyde derivatives such as benzaldehyde; or a combination thereof.

### Other Miscellaneous Additives

Other suitable ingredients include colorants, preservatives (such as methyl and propyl parabens, for example), and rheology modifiers (such as silicon dioxide, for example). Rheology modifiers modify the rheological properties such as viscosity, elasticity, and or yield stress. The colorants, preservatives, and rheology modifiers may be present at levels of from about 0.1%, 0.2, 1, 2, 5, to about 1, 5, 10, 20%, or any combination. Additionally, the compositions may also comprise one or more solvents. These optional solvents may be miscible with the viscosity index improver, water insoluble component, or both, and/or be capable of being dissipated in-situ.

### ORAL CARE COMPOSITION

The oral care composition can take many different forms. For example, the composition can be an emulsion, dispersion, slurry, gel, cream, paste, solid, preformed, or combinations thereof. In one embodiment, the oral care composition is in the form of a gel, cream, paste, wafer, or strip. In another embodiment, the oral care composition can be dispensed from a tube.

The adhesive composition also has many properties. In one embodiment, the composition is bioerodible, non-aqueous, or a mixture thereof. In some embodiments the composition of the present invention erodes. In another embodiment, the oral care composition is preformed. In a further embodiment, the preformed oral care composition further comprises a backing layer.

### Backing Layer

The oral care compositions may be provided as a standalone film or may be applied to, coated on, or otherwise provided with a backing layer. The backing layer can be provided as a single layer or as a laminate formed from a plurality of layers, such as any combination of foam, mesh, and/or other suitable material. The backing layer can be water permeable, water impermeable, partially water permeable, water soluble, water insoluble, erodible, or a combination thereof. Additionally, the backing layer can be continuous or discontinuous (for example, formed from a plurality of discrete segments).

In one embodiment, the backing serves as a protective barrier for the adhesive and/or active. The barrier prevents substantial leaching and/or erosion of the adhesive and/or active by, for example, the wearer's lips, tongue, cheek, as well as saliva. This allows the active in the oral care composition to act upon the oral surface for an extended period of time, from several minutes to several hours. The term "act upon" is herein defined as bringing about a desired change. For example, if the oral care composition is an anti-microbial substance, it reduces or eliminates proliferation of microbial growth that has an overall positive impact on the oral cavity including teeth and gingival tissue.

The backing may comprise polymers, natural and synthetic woven materials, non-woven material, foil, paper, rubber, and combinations thereof. The backing may be a single layer of material or a laminate of more than one layer. Preferably, the material is any type of polymer or combination of polymers that have flexural rigidity and are compatible with oral care substances. Suitable polymers include, but are not limited to, polyethylene, ethylvinylacetate, polyesters, ethylvinyl alcohol and combinations thereof. The shape of the backing is any shape and size that covers the desired oral surface.

### Release Liner

The oral care compositions may also further comprise a release liner. The release liner may be formed from any material which exhibits less affinity (including zero affinity) for the oral care composition than the oral care composition exhibits for itself and for the backing. In one embodiment, the release liner comprises a rigid sheet of material such as polyethylene, paper, polyester, or other material which is then coated with a non-stick type material.

The oral care composition and its components may contain any combination of elements and properties as disclosed herein.

### METHODS OF MANUFACTURE

Oral care compositions can be manufactured by several methods. One example of method for manufacturing includes: a) adding a viscosity index improver and/or water insoluble component to a vessel, b) heating and mixing the viscosity index improver and/or water insoluble component to at least about 40°C, and c) adding and mixing the adhesive component and the oral care active. The order of addition of the components is not believed to be critical so long as the adhesive component is present within the composition when the viscosity index improver and/or water insoluble component are substantially in liquid form. The temperature of the method will need to be adjusted based on the requirements for the viscosity index improver and/or water insoluble component being used. Additionally, the degradation by heat of the oral care active being used should also be considered in determining when to add the active to the composition.

The preformed compositions herein can be formed by processes conventional in the arts, e.g. the film making industries such as casting, coating, calendaring, and extrusion. In one embodiment the separate components of the composition are melted and then blended in a mixing tank until a homogeneous mixture is achieved. Thereafter, the melted mixture may be cast to an acceptable thickness, on an appropriate substrate. Examples of such substrates include Mylar, continuous moving stainless steel belt (which may eventually entering a dryer section if needed), release paper and the like. The compositions are then cooled. The compositions may then be dried if needed, e.g. in a forced-air oven. The temperature of the drying air and length of drying time depend on the nature of the solvent utilized as is recognized in the art. Generally, the drying temperatures include a temperature between about 25°C and 140°C, in another embodiment from about 60° and 90° C for a duration of about 20 minutes to about 60 minutes, in another embodiment from about 30 to about 40 minutes. The composition may then be cut into desired shapes with desired dimensions and then stacked and/or subsequently packaged.

Another conventional film-making process known in the art is extrusion. This method is possible with films wherein the film forming ingredient comprises a variety of extrudable materials. The mechanical particulars of the extrusion process, e.g. the particular equipment utilized, the extruding force, the shape and temperature of the orifice and/or dies are considered to be within the skill of the art and can be varied in a known manner to achieve the physical characteristics of the preformed oral care compositions described herein.

In one embodiment the thickness of the preformed compositions herein is generally between about 0.1 mm to about 2.5 mm, in another embodiment is from about 0.4 mm to about 1.5 mm thick, in another embodiment is from about 0.5 mm to about 1mm thick. The composition may be thicker or thinner depending on the degree of cushioning desired by the user.

### COMPOSITION USE

The present compositions are generally applied to a portion of the oral cavity. Additionally, the oral care compositions can be used in humans and animals. In one embodiment, the oral care composition is used on a household pet. In a further embodiment, the household pet is a mammal comprising a dog. According to another embodiment, the oral care composition is used on a human.

The compositions can be applied alone or in combination with a backing and/or a release liner. When applied to the oral cavity, the oral care compositions can be used, for example, as a bandage to help protect a wound from infection. In another embodiment, the oral care composition can be used as a wound closure. Additionally, in other embodiments, the oral care composition can be used to treat the oral cavity. For example, in one embodiment, an oral care
composition can be used to treat inflammation, halitosis, an infection, a burn, or a combination thereof. In another embodiment, an oral care composition can be used, for example, to deliver a vitamin, mineral, or other beneficial active. Moreover, in an additional embodiment, an oral care composition can be used to deliver a flavorant. Thus, as can be seen from the above, the oral care compositions can be used, for example, in methods to treat varying ailments and conditions of an oral cavity, methods to deliver an active to at least a portion of an oral cavity, methods to deliver actives for systemic use, etc.

The composition may be applied to any suitable location on the oral cavity. In one embodiment the oral car composition wearer generally wears the composition from about 1 hour to about 3 days, in another embodiment from about 6 hours to about 24 hours. After usage, in one embodiment, the oral care composition may be removed from the oral cavity, and any remaining composition may be cleaned from the oral cavity, for example, by gentle scrubbing with water and a brush.

### TEST METHODS

### Procedure to Prepare the Reference Sample (RS) and Prototype Sample (PS)

The reference sample is considered the standard and is made using the standard water insoluble components, while the prototype sample is made using the viscosity index improver being tested.

### MATERIALS

1. Standard Denture Adhesive Components and Excipient Powders (to prepare samples of both the RS and PS):
   i. Ca(47.5)/Zn(17.5) MVE/MA (Methyl Vinyl Ether/ Maleic Acid) mixed partial salt (33%)
   ii. Sodium Carboxymethylcellulose (20%)
   iii. Colloidal Silicon Dioxide (1.14%)
2. Water Insoluble Components (WIC) and Viscosity Index Improver
   iv. To prepare a sample of the RS using standard WIC:
      - Mineral Oil (Drakeol 35 from Penreco) (23.95%) + White Petrolatum ("Snow" from Penreco) (21.91%)
      OR
      To prepare a sample of the PS using the prototype viscosity index improver and WIC:
      - Mineral Oil (Drakeol 35 from Penreco) (40.812%) + Prototype viscosity index improver (5.048%)

### PROCEDURE

The Reference Sample and Prototype Sample are both prepared using the following procedure:
Connect a mixer with wall-scraper blades (Unimix from Haagen and Rinau) and hot water jacket to a water bath and a vacuum pump. Set the water bath of the hot water jacket to about 95°C. Add the WIC and/or viscosity index improver ingredients to mixer vessel. If the water insoluble component and/or viscosity index improver are not liquid at room temperature, allow them to soften before turning on the agitator. Turn on the agitator to about 60 RPM; mix the WIC and/or viscosity index improver ingredient(s) until their temperature reaches about 95°C. Add the "Standard Denture Adhesive Components and Excipient Powders" via a funnel to the mixer with the vent open. Close the vent and stop mixing. Scrape off powder clumps. Re-start mixing at about 60 RPM. Pull about 24 inches Hg vacuum and mix until the batch reaches about 90°C. Reduce bath temperature to about 60°C and continue mixing under vacuum until the batch reaches about 65°C. Stop mixing, turn off the pump, slowly open the vent, release the vacuum, and raise the lid. Fill the sample into a suitable container, such as a foil tube of about 1.4 oz in capacity. Allow samples to equilibrate for about one week. Just prior to testing, squeeze out and discard approximately the first 2 grams from the tube(s).

Whenever possible, the RS and PS are made with the same denture adhesive components and excipient powders at the same levels and with the same manufacturing procedure. This is done to provide a standard matrix to test the differences between a variety of viscosity index improvers by keeping all other variables including the denture adhesive components and sample preparation procedure the same. Among other properties imparted by the standard denture adhesive components, they also provide a standard driving force for the saliva and moisture to penetrate through the denture adhesive composition, and also provide a standard matrix to test the effect of a variety of viscosity index improvers.

If it is necessary to accommodate any property of the prototype viscosity index improver that is not accommodated by the process detailed above (for example if it softens only at temperatures greater than 95°C), the processing temperature profile can be modified as needed.
Similarly, if the above blend of standard denture adhesive components is not suitable, then, just a single denture adhesive component, for example, sodium carboxymethylcellulose at 53%, can be used instead of the blend with Ca/Zn MVE/MA salt. Additionally, if the above testing formulation gives a PS which is too thick to test for the instant viscosity ration as described below, then the sample may need to be diluted with additional water insoluble component like mineral oil.

The above process tests for viscosity index improvers at a level of about 5%. It is believed that testing the prototype viscosity index improvers at 5% will help set-up a baseline, meaning that a finding of viscosity index improver properties at a level of 5% is indicative of viscosity index improver properties at high levels. That being said, a prototype viscosity index improver which is tested at 5% and is found not to have viscosity index improver properties at that level may have them at a higher percentage and should be tested at a higher level to confirm.

The above process can also be scaled up and used for general manufacturing at the temperature appropriate for the viscosity index improver and/or water insoluble component of the denture adhesive composition.

### Instant Viscosity Ratio Test

The Instant Viscosity Ratio can be measured and calculated by the following procedure:

### Equipment:

▪ Ares Strain-Controlled Rheometer
▪ 25 mm permanent parallel plates

### Method:

1. Load 25 mm parallel plates onto an Ares rheometer.
2. Zero the normal force.
3. Zero the gap @ 25°C (i.e. room temperature).
4. Apply the sample to the bottom plate in a semi circular motion moving across the plate. There should be enough specimen such that when a gap of 2.177 ± 0.005 mm is reached and excess is trimmed, the specimen extends evenly to all edges of the plate with no gaps present.
5. Adjust the Gap using the following procedure:
   - Click on set gap icon. Set command gap position to 2.55mm.
   - Set the Max Force Allowed to 100 g.
   - Click on set Gap.
   - Trim sample with plastic cover slide.
   - Set the command gap position to 2.177 mm, Max Force Allowed = 100g.
   - Click on set Gap.
   - Trim sample with plastic cover slide.
   - Set command gap position to 2.147 mm. Max Force Allowed = 100g.
   - Click on set Gap.
   - Do Not Trim Sample.
   - Final Gap should read 2.147 ± 0.005mm
   - Allow the temperature to equilibrate to 25°C.
   - Record the Gap and the Axial Force in test notes along with any observations made.
   - Start Experiment
6. Start test:
   - Method is a Step Rate (Transient) test that runs the following procedure:
      i. Applies a rate of 0/s for 1 s (a 1 s delay)
      ii. Applies a rate of 5/s for 5 s
   - Result should be a curve of Viscosity vs. Time
7. Record the peak viscosity (aka "Instant Viscosity") of this curve.
8. Repeat steps 1-7 for the PS at 25°C - a minimum of three times
9. Repeat steps 1-7 for the PS at 40°C - a minimum of three times
10. Calculate the average value of the Instant Viscosity for the PS at 25°C, and separately at 40°C.
11. Finally, calculate
   - "Instant Viscosity Ratio" = (Average Instant Viscosity for the Composition at 40°C) / (Average Instant Viscosity for the Composition at 25°C)

The following examples further describe and demonstrate embodiments within the scope of the present invention. The examples are given solely for the purpose of illustration and are not to be construed as limitations of the present invention. Many variations of these are possible without departing from the spirit and scope of the invention.

### EXAMPLES

The following are some non-limiting examples of certain embodiments of the present invention.

| **Example I A-Tooth Gels** | A | B | | **Example I B- Tooth Gels** | A | B | C |
|---|---|---|---|---|---|---|---|
| | % | % | | | % | % | % |
| **Part A** | | | | Carbamide Peroxide | 10.00 | 0.00 | 0.00 |
| Zeodent Z119 | 20.00 | 20.00 | | Zeodent Z119 | 0.00 | 0.00 | 20.00 |
| Sodium Polyphosphate | 10.00 | 10.00 | | Zinc Lactate | 0.00 | 2.00 | 0.00 |
| Sodium Saccharin | 2.0 | 2.00 | | Erythritol | 0.00 | 0.00 | 0.00 |
| Poloxamer | 5.00 | 5.00 | | Flavor(s) | 2.00 | 2.00 | 2.00 |
| Powdered Sodium Lauryl Sulfate | 2.00 | 0.00 | | Saccharin | 0.50 | 0.50 | 0.50 |
| **Part B** | | | | PEG 40-Hydrogenated Castor Oil | 10.00 | 10.00 | 10.00 |
| Flavor(s) | 8.00 | 4.00 | | Glycerin | 30.00 | 30.00 | 30.00 |
| Dye | 0.50 | 0.50 | | Petrolatum | 47.50 | 55.50 | 37.50 |
| Polyethylene Speckles | 0.50 | 0.50 | | | | | |
| Petrolatum | 52.00 | 58.00 | | | | | |

The compositions of Example I A are prepared using the following method: 1) melt the petrolatum in an oven set at 65°C, 2) add all powders of Part A in a pestle and mortar and mix, 3) add flavor and dye into the molten petrolatum at lowered temperature and mix with a spatula, 4) add the powder blend from step 2 into the molten petrolatum and mix it into the petrolatum with a spatula, and 5) mix at 1000 RPM for 2 minutes then remove and scrape away materials from the walls with a spatula.

The compositions of Example I B are prepared using the following method: 1) melt the petrolatum in an oven set at 65°C, 2) add all powders in the glycerin and disperse, 3) add all liquids, suspensions, and PEG 40-hydrogenated castor oil into the molten petrolatum and mix with a spatula, and 4) mix at 1000 RPM for 2 minutes then remove and scrape away materials from the walls with a spatula.

The compositions of Example I are examples of oral care compositions with an adhesive component, water insoluble component, and an oral care active. The compositions of Example I can be used as tooth gels. They can be brushed or spread onto all or part of the oral cavity and will provide long lasting benefits. The compositions of Example I A can provide anti-tartar benefits as well as a clean mouth feel, and fresh breath. Composition A of Example I B can provide tooth whitening, anti-microbial benefits, and breath freshening. Composition B of Example I B can provide anti-microbial benefits and breath freshening. Composition C of Example I B can provide a clean mouth feel and breath freshening.

### Example II A- Dental Composition

| | A | B | C | D |
|---|---|---|---|---|
| | % | % | % | % |
| Zinc Lactate Dihydrate (milled) | 0.00 | 12.00 | 0.00 | 0.00 |
| CMC | 52.00 | 41.00 | 41.00 | 41.00 |
| Zinc Carbonate | 0.00 | 0.00 | 12.00 | 0.00 |
| CPC | 1.00 | 0.00 | 0.00 | 0.00 |
| Zinc Citrate Trihydrate | 0.00 | 0.00 | 0.00 | 12.00 |
| Wax W835 | 47.00 | 47.00 | 47.00 | 47.00 |

### Example II B- Dental Composition

| | A | B | C | D | E |
|---|---|---|---|---|---|
| | % | % | % | % | % |
| Stannous Fluoride | 2.00 | 0.00 | 0.00 | 0.00 | 0.00 |
| CMC | 51.00 | 41.00 | 35.00 | 54.00 | 48.20 |
| Carbamide Peroxide | 0.00 | 12.00 | 18.00 | 0.00 | 0.00 |
| Wax W835 | 47.00 | 47.00 | 47.00 | 36.00 | 47.00 |
| Flavor(s) | 0.00 | 0.00 | 0.00 | 4.00 | 2.00 |
| Saccharin | 0.00 | 0.00 | 0.00 | 2.00 | 0.80 |
| Menthol | 0.00 | 0.00 | 0.00 | 4.00 | 2.00 |

The oral care compositions of Example II are prepared by the following procedure: 1) melt the wax in an oven set at 90°C, 2) shake the powder items in a jar to blend them together and eliminate lumps, 3) add the powder blend from step 2 into the molten wax and blend it into the wax with a spatula, 4) mix at 1000 RPM for 2 minutes and scrape the materials from the walls with a spatula, and 5) mix for an additional 4 minutes. The sample is then cooled and extruded into a sheet and cut into small dots, say 1 cm in diameter.

The oral care compositions of Example II are preformed. The preformed compositions are capable of being placed within the oral cavity, for example, on the teeth. For those applications where a localized treatment is desired, for example, an anti-microbial benefit, the oral care composition will be placed in the area where treatment is desired. Composition A from Example II A can provide anti-microbial benefits, anti-gingivitis benefits, and anti-malodor benefits. Compositions B - D from Example II A can provide anti-microbial benefits. Composition A from Example II B can provide anti-microbial benefits, anti-gingivitis benefits, and anti-cavity benefits. Compositions B and C from Example II B can provide tooth whitening, anti-microbial benefits, and breath freshening. Compositions D and E can provide clean mouth feel and breath freshening.

### Example IIIA - Erodible Strips

| | A | B | C | D | E |
|---|---|---|---|---|---|
| | % | % | % | % | % |
| CMC | 12.00 | 0.00 | 10.00 | 20.00 | 40.00 |
| Carbamide Peroxide | 12.00 | 12.00 | 12.00 | 12.00 | 12.00 |
| Wax W835 | 24.00 | 12.00 | 78.00 | 68.00 | 48.00 |
| Wax W445 | 47.00 | 41.00 | 0.00 | 0.00 | 0.00 |
| Sorbitol | 0.00 | 30.00 | 0.00 | 0.00 | 0.00 |
| Saccharin | 2.00 | 2.00 | 0.00 | 0.00 | 0.00 |
| Flavor(s) | 3.00 | 3.00 | 0.00 | 0.00 | 0.00 |

### Example IIIB - Erodible Strips

| | F | G | H | I | J |
|---|---|---|---|---|---|
| | % | % | % | % | % |
| CMC | 10.00 | 20.00 | 40.00 | 40.00 | 40.00 |
| Carbamide Peroxide | 12.00 | 12.00 | 12.00 | 12.00 | 12.00 |
| Wax W835 | 0.00 | 0.00 | 0.00 | 43.00 | 0.00 |
| Wax W445 | 78.00 | 68.00 | 48.00 | 0.00 | 43.00 |
| Sorbitol | 0.00 | 0.00 | 0.00 | 0.00 | 0.00 |
| Saccharin | 0.00 | 0.00 | 0.00 | 2.00 | 2.00 |
| Flavor(s) | 0.00 | 0.00 | 0.00 | 3.00 | 3.00 |

The oral care compositions of Example III are prepared using the following procedure: 1) melt the wax(es) in an oven set at 90°C, 2) mortar and pestle the powder items individually, 3) shake all of the powder items except the carbamide peroxide in jar to blend them together taking care of any lumps with a spatula, 4) add the flavor oils into the molten wax and mix with a spatula, 5) add the powder blend from step 3 into the molten wax and mix it into the wax with a spatula, 6) mix at 1000 RP for 2 minutes and then scrape away any materials from the walls with a spatula, 7) add the carbamide peroxide at a temperature below about 60°C and mix it into the wax with a spatula, 8) mix in the mixer for an additional 4 minutes, 9) allow the mixture to cool overnight, 10) extrude the mixture into 0.7 mm thick strips once the mixture has reached room temperature, and 11) die cut the mixture into the desired shape, for example, disks of 1 cm diameter or strips of 1.5 cm x 7.5 cm size.

The oral care compositions of Example III are in the form of erodible strips. These compositions can be applied to the oral cavity. The compositions of Example III can be applied to at least one tooth and allowed to bioerode over time to provide their benefits. The benefits provided can include tooth whitening, anti-microbial benefits, and breath freshening.

### Example IV - Oral Gel

| | A | B |
|---|---|---|
| | % | % |
| Sodium Lauryl Sulfate | 7.00 | 7.00 |
| Glycerin | 30.00 | 30.00 |
| Water | 0.00 | 10.00 |
| Mixed Mint | 1.00 | 1.00 |
| Menthol | 1.00 | 1.00 |
| Saccharin | 1.00 | 1.00 |
| Carbomer | 0.50 | 0.50 |
| Polyox | 2.00 | 2.00 |
| Silica Z119 | 15.00 | 15.00 |
| Dye | 0.10 | 0.10 |
| Petrolatum | 42.40 | 32.40 |

The oral care compositions of Example IV are prepared by the following method: 1) mix the powders with a mortal and pestle, 2) disperse the powders in the glycerin, mix all of the liquids, 3) add the liquids to the powders and mix with a spatula, and 4) mix the mixture at 1000 RPM for 2 minutes.

The oral care compositions of Example IV are in the form of an oral gel. These compositions may be brushed onto the oral cavity, for example the teeth, and can provide a clean mouth feel and breath freshening.

Polyethylene AC 6702 can be substituted for the microcrystalline wax in the above examples.

The components of the examples may be mixed together to provide hybrid compositions and benefits.

All documents cited in the Detailed Description of the Invention are, in relevant part, incorporated herein by reference; the citation of any document is not to be construed as an admission that it is prior art with respect to the present invention.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

## Claims

1. An oral care composition, comprising:
a) an adhesive component,
b) a water insoluble component, wherein the water insoluble component is petrolatum, and
c) an oral care active, wherein the active is a whitening agent and wherein the composition is adapted to be applied to at least a portion of the oral cavity and wherein the oral care composition is not a denture adhesive.

2. The oral care composition of claim 1, wherein the adhesive component is in an amount from about 10.0% to about 60.0% by weight of the oral care composition.

3. The oral care composition of claim 2, wherein the water insoluble component is in an amount from about 30% to about 70%.

4. The oral care composition of claim 3, further comprising a water insoluble component.

5. The oral care composition of claim 4, wherein the water insoluble component comprises petrolatum, polyvinyl acetate, natural oils, synthetic oils, fats, silicone, silicone derivatives, dimethicone, silicone resins, hydrocarbons, hydrocarbon derivatives, polybutene, oleic acid, stearic acid, essential oils, caprilic/capric triglycerides, or mixtures thereof.

6. The oral care composition of claim 4, wherein the composition is dispensable from a tube.

7. The oral care composition of claim 6, comprising further a viscosity index improver, wherein the viscosity index improver is in an amount from about 0.001 % to about 10.0 % by weight of the oral care composition.

8. The oral care composition of claim 7, wherein the adhesive component is in an amount from about 20.0 % to about 55.0 % by weight of the oral care composition.

9. The oral care composition of claim 8, wherein the water insoluble component is in an amount from about 20% to about 70% by weight of the oral care composition.

10. The oral care composition of claim 3, wherein the composition is preformed.

11. The oral care composition of claim 10, wherein the viscosity index improver is in an amount from about 40 % to about 60 % and the adhesive component is in an amount from about 30% to about 50%.

12. The oral care composition of claim 3, wherein the active selected from the group consisting of anti-microbial, anti-cavity, anti-plaque, anti-tartar, anti-fungal, anti-malodor, anti- inflammatory, anti-erosion, whitening, moisturizer, breath-freshener, anti-gingivitis, antisensitivity, anti -periodontitis, anti -parasitic, and mixtures thereof.
